Europäisches Patentamt

European Patent Office

Office européen des brevets

(19)

(11) Veröffentlichungsnummer: **0 310 952 B1**

(12) **EUROPÄISCHE PATENTSCHRIFT**

(45) Veröffentlichungstag der Patentschrift: **03.06.92**

(51) Int. Cl.⁵: **C07C 409/24**, C12P 1/00

(21) Anmeldenummer: **88116150.9**

(22) Anmeldetag: **30.09.88**

---

(54) **Verfahren zur Herstellung verdünnter Percarbonsäurelösungen.**

---

(30) Priorität: **08.10.87 DE 3734047**
**29.02.88 DE 3806403**

(43) Veröffentlichungstag der Anmeldung:
**12.04.89 Patentblatt 89/15**

(45) Bekanntmachung des Hinweises auf die
Patenterteilung:
**03.06.92 Patentblatt 92/23**

(84) Benannte Vertragsstaaten:
**AT BE CH DE ES FR GB GR IT LI LU NL SE**

(56) Entgegenhaltungen:
**EP-A- 0 214 569**
**GB-A- 1 401 312**
**US-A- 3 169 986**

(73) Patentinhaber: **Henkel Kommanditgesellschaft auf Aktien**
**Postfach 1100 Henkelstrasse 67**
**W-4000 Düsseldorf 1(DE)**

(72) Erfinder: **Weiss, Albrecht, Dr.**
**Forellenweg 37**
**W-4018 Langenfeld-Richrath(DE)**

---

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein katalytisches Verfahren zur Herstellung verdünnter Percarbonsäurelösungen.

Percarbonsäuren sind chemisch recht unstabile Verbindungen, die in reiner Form schwer hergestellt und gelagert werden können. Sie haben dennoch zahlreiche Anwendungen gefunden, so beispielsweise als Bleichmittel bei Temperaturen unterhalb von 100 °C sowie auch als Desinfektionsmittel.

Percarbonsäuren können aus Carbonsäuren und Perhydrol (Wasserstoffperoxid) hergestellt werden, doch verläuft die Bildungsreaktion sehr langsam und bedarf der Katalyse.

Bekanntlich ist die Katalyse durch Enzyme besonders schonend, so daß bereits in der DE-A-22 40 605 vorgeschlagen wird, die Bildung von Percarbonsäuren aus Carbonsäureestern und Wasserstoffperoxid in Gegenwart von Hydrolasen zu bewerkstelligen. Als Hydrolasen werden in der genannten deutschen Patentanmeldung eine Reihe von tierischen und pflanzlichen Lipasen vorgeschlagen. Trotz der Verwendung von Enzymen ist das genannte Verfahren recht unspezifisch, das heißt, aus den eingesetzten Carbonsäureestern entstehen neben vergleichsweise geringen Mengen an Percarbonsäuren hauptsächlich Dicarbonsäuren an sich. Diese geringe Spezifität macht sich insbesondere dann bemerkbar, wenn Carbonsäureester mittlerer Länge (um C8) eingesetzt werden. Gerade Percarbonsäuren dieser Kettenlänge sind jedoch für die Textilbleiche von Interesse.

Die Erfinder haben sich daher die Aufgabe gestellt, ein Enzymsystem mit höherer Selektivität für die Bildung von Percarbonsäuren aus Carbonsäureestern vorzuschlagen. Dabei hat sich überraschend gezeigt, daß Proteasen, das heißt Enzyme, die Amidbindungen angreifen, hier besonders geeignet sind.

Gegenstand der Erfindung ist somit ein Verfahren zur Herstellung von verdünnten Percarbonsäurelösungen durch Umsetzung von Carbonsäureestern mit Perhydrol in Gegenwart von Hydrolasen, dadurch gekennzeichnet, daß als Hydrolase eine Protease eingesetzt wird, und daß gewünschtenfalls in Gegenwart von Tensiden und im Alkalischen gearbeitet wird.

Das erfindungsgemäße Verfahren wird in wässriger Lösung durchgeführt. Die einzelnen Bestandteile wie Carbonsäureester, Wasserstoffperoxid (Perhydrol) oder gewünschtenfalls die Wasserstoffperoxid liefernden Verbindungen, die Enzyme und gewünschtenfalls die weiteren Hilfsstoffe können dabei gelöst oder dispergiert vorliegen.

Nach einer bevorzugten Ausführungsform der Erfindung werden als Proteasen Proteasen von Typ Subtilisin Carlsberg eingesetzt. Geeignete Proteasentypen sind Enzyme, die mit dem Handelsnamen Alkalase ® 2,0 T (Firma Novo), Maxatase ® CX (Firma Gist-Brocades) und P 300 (Firma Biozym) im Handel erhältlich sind. Auch mit gereinigtem Subtilisin Carlsberg werden gute Ergebnisse erhalten. Die Enzymkonzentrationen, bezogen auf proteolytische Aktivitäten nach HPE-Definition und errechnet nach der EPE-Methode, betragen zwischen 100 und 10.000 EPE-Einheiten/ml. Bei einer Esterkonzentration von 5 Gew.-%, bezogen auf Gesamtansatz, haben sich Konzentrationen zwischen 1.000 und 5.000 EPE/ml besonders bewährt.

Unter Proteasen vom Typ Subtilisin Carlsberg werden hier außer Subtilisin Carlsberg an sich auch alle solche proteolytische Enzyme verstanden, die von Subtilisin Carlsberg-Antikörpern als Antigen erkannt werden.

Weiterhin können als Proteasen neutrale oder alkalische Proteasen ganz allgemein eingesetzt werden. So können anstelle von Subtilisin Carlsberg insbesondere auch Serin-Proteasen oder Subtilisin BPN- eingesetzt werden.

Es hat sich gezeigt, daß für die Verwendbarkeit der Enzyme deren Herkunft wenig ausschlaggebend ist. So sind Proteasen der genannten Art aus Bakterien, Pilzen oder auch Säugetieren geeignet.

Sehr gute Ergebnisse wurden mit den Säugetierproteasen Chymotrypsin und Trypsin erzielt. Die Enzymkonzentration, bezogen auf proteolytische Aktivitäten nach HPE-Definition und errechnet nach der EPE-Methode, betragen zwischen 100 und 10.000 EPE-Einheiten/ml. Bei einer Esterkonzentration von 5 Gew.-%, bezogen auf Gesamtansatz, haben sich Konzentrationen zwischen 1.000 und 5.000 EPE/ml besonders bewährt.

Als Ausgangsstoffe für die Percarbonsäuren werden im erfindungsgemäßen Verfahren Ester von Monocarbonsäuren eingesetzt. Besonders vorteilhaft sind Ester von Monocarbonsäuren mit 1 bis 24 C-Atomen im Säureteil, insbesondere mit 4 bis 10 C-Atomen im Säureteil.

Im Alkoholteil tragen die Ester vorzugsweise 1 bis 4 C-Atome. Dabei sind Ester von Monoalkoholen besonders bevorzugt.

Da Percarbonsäuren einer C-Kettenlänge um C-8 besonders günstige Eigenschaften als Bleichmittel aufweisen, hat es sich für derartige Anwendungen besonders bewährt, Carbonsäureester der Kettenlänge um C-8, so beispielsweise Octansäuremethylester einzusetzen. Dabei kann der Ester im System teilweise gelöst oder auch dispergiert vorliegen. Eine bevorzugte Esterkonzentration liegt zwischen 1 und 10 Gew.-

EP 0 310 952 B1

%, vorzugsweise um 5 Gew.-%, bezogen auf Gesamtansatz.

Bei dem erfindungsgemäßen Verfahren wird vorzugsweise der pH auf Werte zwischen 8 und 11 geregelt. In manchen Fällen kann es gewünscht werden, in Gegenwart eines Puffersystems zu arbeiten. Zwar kann auch bei höheren pH-Werten gearbeitet werden, doch tritt dabei vermehrt die Hydrolyse des Esters zur freien Carbonsäure auf. Außerdem besteht die Gefahr, daß die Enzymaktivitäten nachlassen oder gar das Enzym denaturiert. Ein Nachlassen der Enzymaktivität ist auch bei niederen pH-Werten zu befürchten.

Die Konzentration an Wasserstoffperoxid beträgt im erfindungsgemäßen Verfahren vorzugsweise zwischen 0,01 und 2 Gew.-% bezogen auf Gesamtansatz. Für viele Anwendungen sind Konzentrationen um 0,1 Gew.-%, etwa zwischen 0,05 und 0,5 Gew.-% hinreichend. Anstelle von Wasserstoffperoxid können auch Verbindungen eingesetzt werden, die beim Auflösen oder durch Hydrolyse Wasserstoffperoxid freisetzen. Derartige Verbindungen sind beispielsweise Alkaliperoxide, Alkaliperborate wie Natriumperborat oder Kaliumperborat, Alkaliperphosphate wie Natriumperphosphat oder Kaliumperphosphat, Alkalipersilikate wie Natriumpersilikat oder Kaliumpersilikat oder Alkalipercarbonat wie Natriumpercarbonat oder Kaliumpercarbonat.

Bei der Durchführung des erfindungsgemäßen Verfahrens können gewünschtenfalls noch weitere Stoffe zugesetzt werden. Für viele Anwendungen ist es insbesondere sinnvoll Tenside zuzusetzen.

Geeignete Tenside sind beispielsweise wasserlösliche Seifen wie die Natrium-, Ammonium- oder Alkylammoniumsalze höherer Fett- oder Harzsäuren mit 8 bis 20 und vorzugsweise 10 bis 18 C-Atomen. Geeignete Fettsäuren können aus Ölen oder Wachsen tierischen oder pflanzlichen Ursprungs wie beispielsweise aus Talg, Schmalz, Kokosöl, Tallöl oder deren Mischungen gewonnen werden. Besonders günstig ist die Verwendung der Natrium- und Kaliumsalze eines Fettsäuregemisches aus Kokosöl und Talg wie beispielsweise Natriumkokosölseifen und Kaliumtalgseifen.

Außerdem können anionische Tenside eingesetzt werden, wie beispielsweise wasserlösliche sulfatierte oder sulfonierte Tenside mit 8 bis 26 und vorzugsweise etwa 12 bis 22 C-Atomen in einer Alkylgruppe oder im Alkylteil einer höheren Acylgruppe, ferner auch Alkylethersulfate, Arylalkylethersulfate, Fettsäureestersulfonate. Als nichtionische Tenside können beispielsweise die Kondensationsprodukte aus Alkylphenolen oder Alkylthiophenolen mit Ethylenoxid oder Ethylenoxidkondensationsprodukten mit beispielsweise höheren Fettalkoholen oder Monoestern von Hexolen Verwendung finden, ebenso Alkylglycoside.

Geeignete amphotere Tenside sind meist wasserlösliche Salze aliphatischer Aminderivate, die mindestens eine kationische Gruppe wie beispielsweise einen nichtquartären Stickstoff oder eine quartäre Ammonium- oder Phosphoniumgruppe, mindestens eine Alkylgruppe mit etwa 8 bis 18 C-Atomen und eine anionische, die Wasserlöslichkeit bedingende Carboxyl-, Sulfo-, Sulfato-, Phosphato- oder Phosphonogruppe im Molekül enthalten. Die Alkylgruppe kann gerade- oder verzweigtkettig und das kationische Atom kann gegebenenfalls Teil eines heterozyklischen Ringsystem sein.

Geeignete polare nichtionische Tenside sind beispielsweise offenkettige aliphatische Aminoxide der allgemeinen Formel $R_1 R_2 R_3 N{-}O$, in der $R_1$ eine Alkyl-, Alkenyl- oder Monohydroxyalkylgruppe mit 10 bis 16 C-Atomen und $R_2$ und $R_3$ jeweils Methyl-, Ethyl-, Propyl-, Ethanol- oder Propanolgruppen bedeuten.

Als kationische Tenside können Diamine der allgemeinen Formel $RNHC_2 H_4 NH_2$, in der R eine Alkylgruppe mit etwa 12 bis 22 C-Atomen bedeutet, oder Verbindungen der allgemeinen Formel $R^{'} CONHC_2 H_4 NH_2$, in der $R^{'}$ eine Alkylgruppe mit etwa 12 bis 18 C-Atomen bedeutet, sowie quarternäre Ammoniumverbindungen Verwendung finden.

Vorzugsweise werden anionische oder nichtionische Tenside, und zwar insbesondere Salze höherer Alkylbenzolsulfonate oder höherer Alkylsulfonate sowie höhere Fettsäuremonoglyceridsulfate, eingesetzt.

Insbesondere hat sich gezeigt, daß das erfindungsgemäße System mit Vorteil in Gegenwart von Natriumdodecylsulfat durchgeführt werden kann. Im Vergleich dazu wurde beobachtet, daß die Aktivität von Lipasen in Gegenwart von Tensiden meist nachläßt.

Das erfindungsgemäße Verfahren eignet sich insbesondere zur Herstellung verdünnter Persäurelösungen, die dann direkt zum Bleichen oder Desinfizieren eingesetzt werden können.

## Beispiele

1. Enzymatische Umsetzung

1.1 Inkubation

Im Standardsystem werden die Substrate Octansäuremethylester (5%v/v), $H_2 O_2$ (1000 ppm) in Pufferlösung mit dem gewünschten pH-Wert vorgelegt (pH 9,0) und die Reaktion mit der Zugabe von Enzymlösung gestartet.

## 1.2 Probennahme

Aus einem Reaktionsgefäß werden im Verlauf der Reaktion mehrere Aliquots entnommen und die Aktivsauerstoffkonzentrationen bestimmt.

## 1.3 Auswertung

Zur Auswertung der Reaktion werden Analyseergebnisse der Hydrolyse und der ermittelten Persäurekonzentration herangezogen.

Im Reaktionsansatz laufen die Kokurrenzreaktionen Hydrolyse und Perhydrolyse ab. Deshalb ist die Selektivität der Persäurebildung ein entscheidendes Kriterium für die Beurteilung des Reaktionssystems. Als Selektivität wurde das prozentuale Verhältnis der gebildeten Persäure zur freigesetzten Octansäure definiert.

Daneben interessiert der absolute Verbrauch der eingesetzten Substrate bzw. der prozentuale Anteil des verbrauchten Substrats.

2. Nachstehend sind die Prinzipien der Analysemethoden beschrieben.

## 2.1 Bestimmung von Octansäure

Die Bestimmung der Octansäure erfolgt titrimetrisch mit 0,05 N NaOH. Der pH-Wert wird während der Reaktion durch eine pH-Stat-Apparatur konstant gehalten. Aus dem Laugenverbrauch wird die Menge der freigesetzten Säuren berechnet (Summe aus Octansäure und Peroctansäure).

## 2.2 Bestimmung von Aktivsauerstoff ($H_2O_2$)

Die Bestimmung von Aktivsauerstoff wird über eine KJ/Thiosulfat-Titration an einem Autotitrator potentiometrisch durchgeführt. Dabei oxidiert das im Reaktionsgemisch vorhandene $H_2O_2$ das zur Analytik zugesetzte Jodid zu Jod. Das gebildete $J_3$-Ion färbt die Reaktionslösung gelb bis dunkelbraun. Die optisch wahrnehmbare Färbung dient als zusätzliche Kontrolle zu den Analyseergebnissen des Autotitrators.

## 2.3 EPE-Methode

Proteolytische Aktivitätsbestimmung über esterolytische Nebenaktivitäten

| | |
|---|---|
| Prinzip: | Alkalische Proteasen spalten phenolische Esterbindungen; Phenolbildung wird gemessen |
| Inkubation: | N-CBZ-Aminosäure-p-Nitrophenylester werden bei 25 °C pH 6,0 in N-CBZ-Aminosäure und p-Nitrophenol gespalten. |
| Quantifizierung: | Die Bildung des p-Nitrophenol wird direkt im Photometer verfolgt und dE/dt direkt ausgedruckt. |
| Auswertung: | Über einen mitbestimmten Standard (Maxatase) |
| Meßbereich: | CBZ-Valin-p Nitrophenylester 380-1600 PE/ml CBZ-Glycin-p Nitrophenylester 30-140 PE/ml |
| Gerät: | Uvikon 810, Fa. Kontron |
| Testprinzip: | CBZ-Aminosäure-p-Nitrophenylester werden in Aminosäuren und p-Nitrophenol gespalten, dessen Konzentration bei 340 nm bestimmt wird. |

3. Bestimmung von Aktivsauerstoff und Ergebnisse (Persäure)

Die Bestimmung der gebildeten Persäure erfolgt indirekt über die Bestimmung des Aktivsauerstoffgehalts der Lösung.

Im Reaktionssystem liegt ein etwa 100facher $H_2O_2$-Überschuß vor, der die Analytik der Persäure nicht beeinflussen darf. Deshalb wird der in der Analysenlösung vorhandene Wasserstoffperoxid unter der Einwirkung des Enzyms Katalase zersetzt.

Anschließend wird der verbleibende Aktivsauerstoff wie oben bestimmt. Im Falle der Bildung von Peroctansäure aus Trioctanoat wurde nachgewiesen, daß über diese indirekte Methode tatsächlich Peroctansäure bestimmt werden kann.

Persäuren werden durch Katalase in der Regel nicht angegriffen. Bei einem positiven Persäurenachweis ist also die gemessene Persäurekonzentration kleiner oder gleich der tatsächlichen Konzentration. Die Wahrscheinlichkeit, daß andere Perverbindungen außer Persäure erfaßt werden, ist vernachlässigbar.

Persäurebildung in u mol - Inkubationszeit: 60 Minuten - pH-Wert: 9,0, Raumtemperatur - Werte bezogen auf 1 l - Enzymkonzentration 2.500 EPE/ml

| | |
|---|---|
| Subtilisin Carlsberg gereinigt | 215 u mol |
| Maxatase | 250 u mol |
| Subtilisin Carlsberg aus B. licheniformis | 240 u mol |
| ohne Enzym | 0 u mol |

| Enzym-Typ/ Handelsname | Hersteller | Proteintyp Ouchterlony-Test | | Proteasetyp | | | Herkunft | | |
|---|---|---|---|---|---|---|---|---|---|
| | | BPN' | S.Carlsb. | alk. | neutr. | Serin | Bakterien | Pilze | Säugetiere |
| Alcalase 2.OT | Novo | | × | × | | × | × | | |
| Fungal | Sturge | | | | | | | × | |
| Subtilisin | Henkel | | | × | | × | × | | |
| Subtilisin | Henkel | × | | × | | × | | | |
| Chymotrypsin | Boehringer | | | | | × | | | × |
| Trypsin | Merck | | | | | × | | | × |
| Optimase | Miles Kali | × | × | | | | | | |
| Subt.Carlsberg | Sigma | | × | × | | | | | |
| Subt.Carlsberg | Henkel | | × | × | | × | × | | |
| ohne Enzym | – | | | | | | | | |

EP 0 310 952 B1

4. Vergleichsversuche

Als Vergleichsversuche wurden Versuche zur Elektrolyse mit Lipasen durchgeführt.

Standardansätze gerührt mit 1 000 ppm $H_2O_2$, 5 % Methyloctanoat, Puffer pH 9, pH-Stat, Raumtemperatur

| Enzym-Typ/ Handelsname | Hersteller | Einsatzkonzentration pro 200 ml Ansatz | | | Verbrauch NaOH umol | | PPM A.O. Persäure | PPM A.O. Persäure |
|---|---|---|---|---|---|---|---|---|
| | | g | g Protein | EPE | T=30 min | T=60 min | T=30 min | T=60 min |
| Alcalase 2.0T | Novo | 1.568 | 0.0488 | 200000 | 109.6 | 211.3 | 2.7 | 4.0 |
| Fungal | Sturge | 1.780 | 0.0488 | – | 67.2 | 132.5 | 0.8 | 0.8 |
| Subtilisin | Henkel | 0.512 | 0.0488 | – | 87.6 | 176.7 | 1.6 | 2.4 |
| Subtilisin | Henkel | 5.787 | – | 200000 | 66.5 | 133.3 | 0.6 | 0.9 |
| Chymotrypsin | Boehringer | 0.175 | 0.0488 | 14612 | 103.3 | 193.7 | 3.3 | 5.7 |
| Trypsin | Merck | 0.700 | 0.0488 | 13130 | 129.7 | 236.2 | 4.2 | 6.4 |
| Optimase | Miles Kali | 0.112 | – | 200000 | 98.7 | 182.2 | 2.7 | 3.8 |
| Subt.Carlsberg | Sigma | 0.266 | – | 200000 | 225.7 | 283.4 | 2.4 | 3.5 |
| Subt.Carlsberg aufgerein. | Henkel | 0.166 | – | 200000 | 223.0 | 316.5 | 2.6 | 3.7 |
| ohne Enzym | – | – | – | – | 41.0 | 90.0 | 0 | 0 |

7

Testsystem:

5 % Methyloctanoat (v/v), 1000 ppm Aktivsauerstoff $H_2O_2$, pH 9,0, pH-Stat-Methode

Lipasetypen:

Lipase P, Fa. Amano, Mikroorganismus: Pseudomonas sp.
Lipase B, Fa. Amano, Mikroorganismus: Pseudomonas nitroreducens
Lipase Steapsin, Fa. Sigman, aus Schweinepankreas gewonnen
Lipase F-AP, Fa. Amano, Mikroorganismus: Rhizopus javanicus
Lipase LP, Fa. Toyo Sanyo, Mikroorganismus: Chromobacterium viscosum

Ergebnisse:

Im Testsystem hydrolysieren sämtliche eingesetzten Enzympräparate den Octansäuremethylester. Die möglicherweise erzeugten Persäuremengen waren in allen Fällen so gering, daß sie nicht nachgewiesen werden konnten.

**Patentansprüche**

1. Verfahren zur Herstellung von verdünnten Percarbonsäurelösungen durch Umsetzung von Carbonsäureestern mit Perhydrol in Gegenwart von Hydrolasen, dadurch gekennzeichnet, daß als Hydrolase eine Protease eingesetzt wird, und daß gewünschtenfalls in Gegenwart von Tensiden und im Alkalischen gearbeitet wird.

2. Verfahren nach Anspruch 1, dadurch gekennzeichnet, daß als Protease alkalische oder neutrale Proteasen eingesetzt werden.

3. Verfahren nach Anspruch 1 oder 2, dadurch gekennzeichnet, daß als Protease alkalische oder neutralische Proteasen aus Bakterien, Pilzen und/oder Säugetieren eingesetzt werden.

4. Verfahren nach einem der Ansprüche 1 bis 3, dadurch gekennzeichnet, daß als Proteasen Trypsin und/oder Chymotrypsin eingesetzt werden.

5. Verfahren nach einem der Ansprüche 1 bis 3, dadurch gekennzeichnet, daß als Protease eine Protease vom Typ Subtilisin Carlsberg eingesetzt wird.

6. Verfahren nach einem der Ansprüche 1 bis 5, dadurch gekennzeichnet, daß als Carbonsäureester Ester von Monocarbonsäuren mit 1 bis 24, vorzugsweise 4 bis 10 C-Atomen eingesetzt werden.

7. Verfahren nach einem der Ansprüche 1 bis 6, dadurch gekennzeichnet, daß als Carbonsäureester die Ester mit Monoalkoholen mit 1 bis 4 C-Atomen eingesetzt werden.

8. Verfahren nach einem der Ansprüche 1 bis 7, dadurch gekennzeichnet, daß bei pH-Werten zwischen 8 und 11 gearbeitet wird.

9. Verfahren nach einem der Ansprüche 1 bis 8, dadurch gekennzeichnet, daß als Tenside Aniontenside in Konzentrationen bis zu 10 Gew.-% zugegen sind.

10. Verfahren nach den Ansprüchen 1 bis 9, dadurch gekennzeichnet, daß Perhydrol in Form einer durch Lösen oder Hydrolyse Perhydrol liefernden anorganischen Perverbindung eingesetzt wird.

**Claims**

1. A process for the production of dilute percarboxylic acid solutions by reaction of carboxylic acid esters with perhydrol in the presence of hydrolases, characterized in that a protease is used as the hydrolase and in that, if desired, the process is carried out in the presence of surfactants and at an alkaline pH value.

**2.** A process as claimed in claim 1, characterized in that alkaline or neutral proteases are used as the protease.

**3.** A process as claimed in claim 1 or 2, characterized in that alkaline or neutral proteases from bacteria, fungi and/or mammals are used as the protease.

**4.** A process as claimed in any of claims 1 to 3, characterized in that trypsin and/or chymotripsin are used as the protease.

**5.** A process as claimed in any of claims 1 to 3, characterized in that a protease of the subtilisin Carlsberg type is used as the protease.

**6.** A process as claimed in any of claims 1 to 5, characterized in that esters of $C_{1-24}$ and preferably $C_{4-10}$ monocarboxylic acids are used as the carboxylic acid esters.

**7.** A process as claimed in any of claims 1 to 6, characterized in that esters with $C_{1-4}$ monoalcohols are used as the carboxylic acid esters.

**8.** A process as claimed in any of claims 1 to 7, characterized in that it is carried out at pH values of 8 to 11.

**9.** A process as claimed in any of claims 1 to 8, characterized in that anionic surfactants in concentrations of up to 10% by weight are present as the surfactants.

**10.** A process as claimed in claims 1 to 9, characterized in that perhydrol is used in the form of an inorganic per compound which yields perhydrol by dissolution or hydrolysis.

**Revendications**

**1.** Procédé de production de solutions diluées d'acides percarboxyliques en faisant réagir des esters d'acides carboxyliques avec du perhydrol en présence d'hydrolases, caractérisé en ce que l'on utilise comme hydrolase une protéase et en ce que, si on le souhaite, on opère en présence d'agents tensioactifs et en milieu alcalin.

**2.** Procédé selon la revendication 1, caractérisé en ce que l'on utilise comme protéases des protéases alcalines ou neutres.

**3.** Procédé selon la revendication 1 ou 2, caractérisé en ce que l'on utilise comme protéases des protéases alcalines ou neutres provenant des bactéries, des champignons et/ou des mammifères.

**4.** Procédé selon l'une des revendications 1 à 3, caractérisé en ce que l'on utilise comme protéases la trypsine et/ou la chymotrypsine.

**5.** Procédé selon l'une des revendications 1 à 3, caractérisé en ce que l'on utilise comme protéases une protéase du type Subtilisine Carlsberg.

**6.** Procédé selon l'une des revendications 1 à 5, caractérisé en ce que l'on utilise comme esters d'acides carboxyliques des esters d'acides monocarboxyliques ayant de 1 à 24 et de préférence de 4 à 10 atomes de carbone.

**7.** Procédé selon l'une des revendications 1 à 6, caractérisé en ce que l'on utilise comme esters d'acides carboxyliques les esters des monoalcools avec de 1 à 4 atomes de carbone.

**8.** Procédé selon les revendications 1 à 7, caractérisé en ce que l'on opère avec des valeurs de pH comprises entre 8 et 11.

**9.** Procédé selon l'une des revendications 1 à 8, caractérisé en ce qu'on ajoute comme agents tensioactifs des tensioactifs anioniques à des concentrations allant jusqu'à 10% en poids au maximum.

9

10. Procédé selon les revendications 1 à 9, caractérisé en ce que le perhydrol est utilisé sous forme d'un percomposé inorganique donnant du perhydrol par dissolution ou par hydrolyse.